## Europäisches Patentamt
## European Patent Office
## Office européen des brevets

(19)

(11) Numéro de publication: **0 185 127**
**B1**

(12) **FASCICULE DE BREVET EUROPÉEN**

(45) Date de publication du fascicule du brevet:
**21.09.88**

(51) Int. Cl.⁴: **A 23 L 2/40**, A 23 L 2/38

(21) Numéro de dépôt: **84810641.5**

(22) Date de dépôt: **19.12.84**

(54) **Procédé de traitement d'acides utilisés comme constituants d'une composition pulvérulente hydrosoluble pour boissons à dégagement gazeux prolongé.**

(43) Date de publication de la demande:
**25.06.86 Bulletin 86/26**

(45) Mention de la délivrance du brevet:
**21.09.88 Bulletin 88/38**

(84) Etats contractants désignés:
**AT BE CH DE FR GB IT LI LU NL SE**

(56) Documents cité:
**DE-A-2 405 659**
**GB-A-1 124 335**

(73) Titulaire: **DRIDRINKS N.V., De Ruyterkade 62, Curaçao N.A. (NL)**

(72) Inventeur: **Lavie, Louis, 6 avenue Dapples, CH- 1006 Lausanne (CH)**

(74) Mandataire: **Vuille, Roman, c/o KIRKER & Cie S.A. 14, rue du Mont- Blanc Case Postale 872, CH- 1211 Genève 1 (CH)**

EP 0 185 127 B1

## 0 185 127

### Description

La présente invention a pour objet un procédé de traitement d'un acide ou mélange d'acides utilisé comme constituant d'une composition pulvérulente pour boissons à dégagement gazeux prolongé.

Le marché des boissons gazeuses, plus particulièrement celui des limonades gazeuses dites "soft drinks" ou "diet drinks", est en progression constante. Sur le plan industriel cependant, son développement se heurte au problème du coût des transports et des manipulations relativement nombreuses d'un produit lourd et volumineux. On tente actuellement de résoudre de tels problèmes en dispersant à l'extrême les centres de production et à ne transporter que des extraits concentrés, à l'état sec ou liquide.

Dans les installations industrielles appropriées, ces extraits sont additionnés d'eau, le cas échéant, de sucre, puis de $CO_2$ sous pression élevée, avant d'être conditionnés dans des récipients hermétiques, résistant à la pression, et de ce fait relativement lourds et encombrants. Le caractère "piquant" de ces boissons est alors assuré, lors de la consommation, par le dégagement progressif de la dose de $CO_2$ initialement dissoute.

On connaît également des extraits secs pulvérulents à base de bicarbonate de sodium et d'acides comestibles qui, additionnés d'eau juste avant consommation, permettent l'obtention de boissons gazeuses. De tels extraits, cependant, ne permettent pas une gazéification de la boisson comparable par sa durée et son "piquant" à celle résultant de la dissolution de $CO_2$ sous pression. On constate en outre que dans de nombreux cas, les ingrédients indispensables à l'effet organoleptique recherché, tel l'acide orthophosphorique, l'acide citrique ou le bicarbonate de sodium par exemple, sont particulièrement hygroscopiques et compromettent de ce fait la stabilité des mélanges conditionnés.

Les industries chimiques et pharmaceutiques produisent et utilisent des polymères pour l'enrobage des médicaments. Certains de ces polymères peuvent convenir pour préserver les extraits secs contre les reprises d'humidité et pour contrôler partiellement la réaction entre les acides et les carbonates. N'ayant pas été conçus pour cet usage, ils présentent des inconvénients tels que la coloration des mousses ou des dépôts dans les verres. Le contrôle et l'uniformisation du dégagement de $CO_2$ dans la boisson ne sont en outre que fort imparfaitement maîtrisés.

L'inventeur a récemment mis au point un procédé d'enrobage conduisant à l'obtention d'une composition pulvérulente dont les constituants, acides et carbonates, sont efficacement protégés contre toute reprise d'humidité et qui, une fois additionnée d'eau, permet la préparation d'une boisson gazeuse dont la gazéification, tant par sa durée que son "piquant", est tout à fait comparable à celle obtenue par le moyen des installations industrielles connues à ce jour.

Ce procédé a fait depuis lors l'objet d'une publication (EP-A-130 144), et pour ce qui est du traitement des constituants acides, le procédé d'enrobage peut être brièvement décrit comme suit: on enrobe l'acide ou le mélange d'acides avec une liqueur obtenue par chauffage en vase clos à 50°C durant au moins 1 heure, en présence d'alcool éthylique, d'une solution aqueuse sucrée d'un polysaccharide macromoléculaire hydrosoluble ou d'un mélange de tels polysaccharides, puis on sèche le produit résultant dans un courant d'air chaud et pulvérise ensuite le produit desséché. Des mises en oeuvre de cette technique antérieure sont détaillées plus loin.

L'invention a pour objet un procédé de traitement d'un acide ou mélange d'acides utilisé comme constituant d'une composition pulvérulente hydrosoluble non hygroscopique destinée à la préparation de boissons à dégagement gazeux prolongé, au cours duquel on traite ledit acide ou mélange d'acides avec une liqueur obtenue par chauffage en vase clos à 50°C durant au moins 1 heure, en présence d'alcool éthylique, d'une solution aqueuse sucrée d'un polysaccharide macromoléculaire hydrosoluble ou d'un mélange de tels polysaccharides, caractérisé en ce qu'on dissout l'acide ou le mélange d'acides et la liqueur dans l'eau, qu'on élimine ensuite l'eau du mélange résultant et que l'on pulvérise ensuite le produit ainsi obtenu.

Par les termes "acide" ou "mélange d'acides" on entend définir, selon la présente invention, un acide ou un mélange d'acides minéral et/ou organique, tel l'acide citrique, tartrique, malique, ascorbique ou orthophosphorique par exemple. Selon l'invention, chacun des ingrédients acides mentionnés ci-dessus peut en outre être mélangé avant traitement, à des substances édulcorantes, naturelles ou synthétiques, telles que sucrose, fructose, saccharine, cyclamates ou aspartame par exemple.

En fonction des effets gustatifs recherchés, lesdits ingrédients acides pourront être également mélangés à des colorants, tel le colorant caramel par exemple, des huiles ou des mélanges d'huiles essentielles, ou divers extraits, tels par exemple la caféine, la quinine, le tolu ou l'extrait soluble de noix de cola.

Selon la présente invention, on utilise un polysaccharide macromoléculaire, telle la gomme arabique ou la gomme adragante, ou encore un mélange de polysaccharides macromoléculaires tels que ceux mentionnés ci-dessus.

Conformément au procédé de l'invention, l'imprégnation des constituants acides s'effectue par le moyen d'une liqueur de saccharose ou de dérivés de saccharose, tel le caramel de sucre par exemple, contenant le ou les polysaccharides macromoléculaires choisis, en proportions prédéterminées.

La composition de la liqueur peut être avantageusement adaptée à la nature des produits à imprégner et à l'effet recherché. En ce qui concerne le groupe des constituants acides, il suffit que l'imprégnation de ceux-ci permette d'obtenir une bonne protection des produits contre l'hygroscopie et un léger ralentissement de la dissolution pour stabiliser le pH en fonction de la durée de la réaction.

Il a été remarqué que les liqueurs à base de gomme arabique conviennent particulièrement bien à l'imprégnation des acides.

2

La liqueur d'imprégnation des acides, en outre, assure une dispersion instantanée des composants dans l'eau du fait de sa dissolution en milieu acide.

Conformément au procédé de l'invention, l'imprégnation des constituants acides est effectuée par dissolution, dans la quantité d'eau minimum nécessaire, des produits et de la liqueur choisis.

De bons résultats ont été obtenus en imprégnant les divers constituants acides à raison de 1,5 à 15 % environ en poids de liqueur par rapport au poids total de mélange à l'état sec, plus généralement à raison de 3 à 7 % environ, sans tenir compte de la quantité d'eau de dissolution des acides.

Une fois les ingrédients choisis convenablement imprégnés, le produit résultant est alors soumis à la dessication et, le cas échéant, réduit en poudre. La dessication peut être convenablement effectuée par séchage sous vide poussé à basse température ou par lyophilisation. Le choix de la méthode de dessication sera fonction de la stabilité et de la volatilité des divers ingrédients. La dessication sera en outre poursuivie de façon à abaisser la teneur en eau du produit imprégné jusqu'à un maximum de 0,5 à 1 % en poids environ.

Si nécessaire, le produit résultant de la dessication est ensuite pulvérisé selon les techniques usuelles, le cas échéant stocké dans l'attente de son utilisation finale.

Chacun des constituants, acides et respectivement carbonates, imprégnés et séchés séparément, est alors mélangé, en proportions prédéterminées, avec le constituant correspondant, et le mélange résultant conditionné sous la forme appropriée à son emploi, de préférence dans un emballage étanche à l'air. Le poids de chacun des groupes de produits à placer dans chaque emballage dépend des éléments qui lui ont été incorporés, mais il doit toujours respecter les proportions fixées entre les acides et le ou les carbonates. La composition pulvérulente ainsi obtenue peut être par exemple répartie en doses unitaires, convenant, après simple addition d'eau, à la préparation de 0,3, 0,5, 1 litre ou plus de boisson gazeuse.

## Applications de la technique antérieure

## Composition pulvérulente pour boisson gazeuse de type "cola"

### 1.1. Préparation des liqueurs d'imprégnation

**Liqueur A:**

On prépare 10 g de caramel de sucre auquel on ajoute 40 g d'eau à 50°C pour liquéfier le caramel.

On mélange au mortier ou au mixer 40 g de sucre pulvérisé et 50 g de gomme arabique.

On dilue le sucre pulvérisé et la gomme arabique avec l'eau tiède contenant le caramel dissous jusqu'à ce que le mélange soit bien unifié et sans grumeaux, puis on ajoute progressivement 30 ml d'éthanol à 95°.

La liqueur ainsi obtenue est ensuite entreposée dans un vase clos placé durant 2 heures dans une étuve chauffée à 50°C.

La liqueur est alors parfaitement unifiée et prête à être employée.

**Liqueur B:**

On prépare 20 g de caramel de sucre auquel on ajoute 50 g d'eau à 50°C pour liquéfier le caramel.

On mélange à sec, au mortier ou au mixer, 40 g de sucre moulu, 40 g de gomme arabique, 10 g de gomme adragante.

On dilue le sucre moulu et les gommes avec l'eau tiède de dilution du caramel à laquelle on ajoute 30 ml d'eau tiède.

Lorsque le produit ne présente plus de grumeaux, on ajoute progressivement 20 ml d'éthanol à 95°, puis la liqueur ainsi obtenue est ensuite entreposée dans un vase clos placé durant 3 heures 30 dans une étuve chauffée à 50°C.

La liqueur est alors parfaitement unifiée et prête à être employée.

Ces liqueurs A et B sont parfaitement stables et se conservent fort longtemps. Etendues en minces pellicules sur des plaques de verre, elles supportent des variations thermiques de -20°C à +50°C sans aucune altération et sans que leur adhérence totale au verre soit modifiée.

### 1.2. Imprégnation des constituants

1) On mélange à sec:

1,437 g colorant caramel pulvérulent
1,890 g acide orthophosphorique moulu
10,720 g acide citrique anhydre
18,900 g acide tartrique moulu
3,000 g extrait de tolu
3,000 g huiles essentielles sur sucre instantané
1,815 g mélange édulcorant (saccharine et cyclamates)

40,762 g

Ce mélange est imprégné avec 2 grammes de liqueur A (5 %), par broyage dans un mortier.

Lorsqu'une imprégnation satisfaisante et homogène est obtenue, le mélange est séché, soit sous vide, soit par le froid, comme il a été dit précédemment.

Le produit desséché est pulvérisé.

2) On mélange à sec:

20,600 g de bicarbonate de sodium
0,595 g de caféine
1,437 g de colorant caramel pulvérulent

22,632 g au total

Ce mélange est imprégné avec 2,26 g de liqueur B (10 %), par broyage au mortier.

Lorsqu'une imprégnation satisfaisante et homogène est obtenue, le mélange est réparti en couches minces sur des toiles métalliques et séché sous un courant d'air chaud à 50°C.

Le produit desséché est pulvérisé.

1.3. Mélange des constituants

Les produits résultant des opérations 1 et 2 ci-dessus sont répartis dans des emballages étanches à l'air, contenant chacun 6,34 g environ d'extrait sec permettant d'obtenir 300 ml de boisson gazeuse de type "cola". Le mélange de 6,34 g est composé de 4,076 g provenant du groupe des acides et de 2,264 g provenant du second groupe.

**Composition pulvérulente pour limonade gazeuse au citron vert**

1) On mélange à sec:

1,89 g d'acide orthophosphorique
10,72 g d'acide citrique anhydre
18,90 g d'acide tartrique moulu
3,00 g de mélange édulcorant (saccharine et cyclamates)
3,00 g d'extrait de tolu
3,00 g d'huile essentielle de limette sur sucre instantané

40,51 g au total

Ce mélange est imprégné à l'aide de 2,30 g de liqueur A (6 %) de façon à obtenir une imprégnation uniforme du produit qui est ensuite desséché sous vide à 30°C.

Après dessication, le produit est pulvérisé.

2) On imprègne 20,60 g de bicarbonate de sodium avec 2 g de liqueur B (selon exemple 1; 10 %).

Le produit ainsi obtenu est séché sous un courant d'air chaud à 50°C.

Les produits résultant des opérations 1 et 2 sont finalement mélangés en proportions adéquates et répartis en doses unitaires logées dans des emballages étanches à l'air. La totalité du mélange ci-dessus permet de préparer 3 l de limonade gazeuse au citron vert.

**Composition pulvérulente pour boisson gazeuse de type "bitter"**

1) On mélange à sec:

1,89 g d'acide orthophosphorique moulu
10,00 g d'acide citrique anhydre
20,00 g d'acide tartrique moulu
2,40 g de mélange édulcorant (saccharine et cyclamates)
0,0015 g de chlorhydrate de quinine

---

34,2915 g au total

Ce mélange est imprégné à l'aide de 1,80 g de liqueur A (6 % environ), dans laquelle on a incorporé 0,30 g d'huile essentielle de citron sur sucre instantané.

La dessication s'opère sous vide partiel à 30°C. Le produit est ensuite pulvérisé.

2) On imprègne:

20,06 g de bicarbonate de sodium à l'aide de 2,00 g de liqueur B (10 % environ).

Le séchage est effectué sous un courant d'air chaud à 50°C.

Le produit sec est pulvérisé.

Après le mélange des produits résultant des opérations 1 et 2, on obtient une composition permettant la préparation de 3 l de limonade gazeuse de type "bitter".

L'invention est illustrée à l'aide de l'exemple ci-dessous. Cet exemple n'est en aucune sorte limitatif.

**Exemple**

**Composition pulvérulente pour boisson gazeuse de type "cola"**

On fait dissoudre dans de l'eau:

1,00 g de colorant caramel
60,00 g de caramel pulvérulent contenant 0,71 g d'acide orthophosphorique (brevet suisse No 625 228)
11,00 g d'acide citrique anhydre
19,00 g d'acide tartrique moulu
3,00 g d'extrait de tolu sur sucre instantané
3,00 g d'huiles essentielles sur sucre instantané
1,80 g de mélange édulcorant (saccharine et cyclamates)
6,00 g d'extrait hydrosoluble de noix de cola

---

104,80 g au total

Pour la dissolution, on a utilisé une quantité d'eau telle que l'on obtienne une solution suffisamment fluide pour être filtrée le cas échéant.

On ajoute à cette solution 3,14 g (3 %) de liqueur A. La solution est agitée en vue de son unification, puis lyophilisée.

Le mélange déshydraté est réduit en poudre.

Le mélange ainsi obtenu peut être ensuite utilisé comme indiqué plus haut pour donner, après incorporation à la quantité correspondante de bicarbonate imprégné de liqueur B, une composition destinée à la préparation d'une boisson gazeuse de type "cola".

**Revendications**

1. Procédé de traitement d'un acide ou mélange d'acides utilisé comme constituant d'une composition pulvérulente hydrosoluble non hygroscopique destinée à la préparation de boissons à dégagement gazeux prolongé, au cours duquel on traite ledit acide ou mélange d'acides avec une liqueur obtenue par chauffage en vase clos à 50°C durant au moins 1 heure, en présence d'alcool éthylique, d'une solution aqueuse sucrée d'un polysaccharide macromoléculaire hydrosoluble ou d'un mélange de tels polysaccharides, caractérisé en ce qu'on dissout l'acide ou le mélange d'acides et la liqueur dans l'eau, qu'on élimine ensuite l'eau du mélange résultant et que l'on pulvérise ensuite le produit ainsi obtenu.

2. Procédé selon la revendication 1, caractérisé en ce qu'on dissout l'acide ou le mélange d'acides dans la quantité d'eau minimum nécessaire.

3. Procédé selon la revendication 1 ou 2, caractérisé en ce qu'on élimine l'eau du mélange résultant par évaporation sous pression réduite.

4. Procédé selon la revendication 1 ou 2, caractérisé en ce qu'on élimine l'eau du mélange résultant par lyophilisation.

5. Procédé selon l'une des revendications 1 à 4, caractérisé en ce qu'on utilise un acide minéral tel l'acide orthophosphorique ou un acide organique tel l'acide citrique, tartrique, malique ou ascorbique.

6. Procédé selon l'une des revendications 1 à 5, caractérisé en ce qu'on utilise au moins un polysaccharide macromoléculaire hydrosoluble choisi parmi la gomme arabique et la gomme adragante.

7. Procédé selon l'une des revendications 1 à 6, caractérisé en ce que l'acide ou le mélange d'acides est traité en présence de substances édulcorantes, de colorants ou de substances à effet organoleptique.


**Patentansprüche**

1. Verfahren zur Verarbeitung einer Säure oder eines Säuregesmisches, verwendet als Bestandteil einer nichthygroskopischen, wasserlöslichen, mehligen Verbindung, die bestimmt ist zum Zubereiten langzeitig kohlensäureabgebender Getränke, im Laufe welchen Verfahrens besagte Säure oder besagtes Säuregemisch mit einer Lösung verarbeitet wird, die man erhält durch Erwärmen bei 50°C während mindestens 1 Stunde in einem geschlossenen Gefäss in Gegenwart eines Äthylalkohols, einer wässrigen Lösung gezuckert mit einem wasserlöslichen, makromolekularen Polysaccharid oder einer Mischung solcher Polysaccharide, dadurch gekennzeichnet, dass die Säure oder das Säuregemisch und die Lösung im Wasser gelöst werden, anschliessend der sich ergebenden Mischung das Wasser entzogen wird und dann das sich so ergebende Gemisch pulverisiert wird.

2. Verfahren nach Patentanspruch 1, dadurch gekennzeichnet, dass man die Säure oder das Säuregemisch in der minimal erforderlichen Wassermenge auflöst.

3. Verfahren nach Patentanspruch 1 oder 2, dadurch gekennzeichnet, dass man das Wasser aus dem sich ergebenden Gemisch durch Verdampfung bei herabgesetztem Druck entfernt.

4. Verfahren nach Patentanspruch 1 oder 2, dadurch gekennzeichnet, dass man das Wasser aus dem sich ergebenden Gemisch durch Lyophilisation entfernt.

5. Verfahren nach einem der Patentansprüche 1 bis 4, dadurch gekennzeichnet, dass man eine Mineralsäure verwendet, wie beispielsweise Phosphorsäure oder eine organische Säure, wie beispielsweise Zitronensäure, Weinsäure, Apfelsäure oder Ascorbinsäure.

6. Verfahren nach einem der Patentansprüche 1 bis 5, dadurch gekennzeichnet, dass man mindestens ein wasserlösliches, makromolekulares Polysaccharid verwendet, und dafür Gummi arabicum und Tragantgummi auswählt.

7. Verfahren nach einem der Patentansprüche 1 bis 6, dadurch gekennzeichnet, dass die Säure oder das Säuregemisch in Gegenwart von Süßstoffen, Farbstoffen oder organoleptischen Substanzen verarbeitet wird.


**Claims**

1. A process for the treatment of an acid or mixture of acids used as a constituent of a non-hygroscopic water-soluble pulverulent composition for the preparation of drinks which release gas over a prolonged period, in which process the said acid or mixture of acids is treated with a liquor obtained by heating a sugar-containing aqueous solution of a water-soluble macromolecular polysaccharide, or a mixture of such polysaccharides, in a closed vessel at 50°C for at least 1 hour, in the presence of ethyl alcohol, characterized in that the acid or mixture of acids and the liquor are dissolved in water, in that the water is then removed from the resulting mixture and in that the product obtained in this way is then pulverized.

2. The process according to Claim 1, characterized in that the acid or mixture of acids is dissolved in the minimum amount of water required.

3. The process according to Claim 1 or 2, characterized in that the water is removed from the resulting mixture by evaporation under reduced pressure.

4. The process according to Claim 1 or 2, characterized in that the water is removed from the resulting mixture by freeze-drying.

5. The process according to one of Claims 1 to 4, characterized in that the acid used is a mineral acid such as orthophosphoric acid, or an organic acid such as citric, tartaric, malic or ascorbic acid.

6. The process according to one of Claims 1 to 5, characterized in that at least one water-soluble macromolecular polysaccharide is used which is selected from gum arabic and gum tragacanth.

7. The process according to one of Claims 1 to 6, characterized in that the acid or mixture of acids is treated in the presence of sweeteners, colours or substances having an organoleptic effect.